# EUROPÄISCHE PATENTANMELDUNG

(11) **EP 4 468 305 A1**
(43) Veröffentlichungstag der Anmeldung: **27.11.2024**
(21) Anmeldenummer: 24174693.2
(22) Anmeldetag: 08.05.2024
(51) Int. Cl.: G16H 30/20, G16H 30/40, A61B 1/04, G06F 11/30, G06F 11/32, H04N 17/02

(54) **MEDIZINISCHE VORRICHTUNG, SYSTEM UND VERFAHREN ZUR ÜBERWACHUNG EINES QUELL-BILDSTROMS**

(30) Priorität: 15.05.2023 DE 102023112796
(71) Anmelder: Karl Storz SE & Co. KG, 78532 Tuttlingen (DE)
(72) Erfinder: Brüderle, Klaus, 78532 Tuttlingen (DE)

(57) **Zusammenfassung**

Vorrichtung (10) zur Überwachung eines Quell-Bildstroms (14) mit mehreren aufeinanderfolgenden Quell-Bildern (16), wobei die Vorrichtung (10) aufweist: eine Verarbeitungseinrichtung (18), die dafür ausgebildet ist, den Quell-Bildstrom (14) zu empfangen, Sicherungswerte (30) zu errechnen, auf den Sicherungswerten (30) basierende Sicherungsinformation (32) den Quell-Bildern (16) hinzuzufügen und die daraus resultierenden Bilder als Sende-Bilder (22) eines Sende-Bildstroms (20) zu senden, und eine Verifikationseinrichtung (24), die dafür ausgebildet ist, den Sende-Bildstrom (20) zu empfangen, Prüfwerte (34) zu errechnen, und die Sicherungswerte (30) gegen die Prüfwerte (34) abzugleichen. Es werden ferner ein entsprechendes System (1) und ein Verfahren (50) aufgezeigt.

## Beschreibung

Die Erfindung betrifft eine medizinische Vorrichtung, ein System und ein Verfahren zur Überwachung eines Quell-Bildstroms mit mehreren aufeinanderfolgenden Quell-Bildern.

Die wichtigste Anforderung im Bereich der medizinische Videoendoskopie ist, Benutzern einen Bildstrom der Endoskop-Kamera zur Betrachtung zur Verfügung zu stellen. Dies geschieht z.B. im Rahmen einer Untersuchung oder einer Operation, indem die Motivszenen live präsentiert oder aufgezeichnet werden. Zusätzlich werden öfter auch begleitende Bild-, Medien- oder Datenströme behandelt.

Bei der heutigen Technik betrifft die Prüfung eines Bildstroms hauptsächlich den Entwicklungsvorgang. Hierbei wird die Plausibilität einer Bildvisualisierung oder Aufzeichnung meisten humanphysiologisch vorgenommen. Konkret prüft ein Tester eine Bildstrom visuell die Aktualität, die Vollständigkeit und die Präsenz von Artefakten über einen entsprechenden Zeitraum.

Manchmal wird im Rahmen eines automatisierten Tests auch ein Testbild eingespeist und am Ziel ausgewertet. Zum Beispiel werden abwechselnd grüne und rote Bilder geschickt und deren Empfang wird geprüft. Dies geschieht in einer dedizierten Testumgebung. Für Worstcase- oder Laufzeit-Aussagen wird dieses dedizierte Testnetz hinsichtlich seiner Leistungsfähigkeit bzw. Performance modifiziert. Eine Zuverlässigkeitsvorhersage über alle unsere Systemkombinationen wird dann auf Basis dieser Ergebnisse aus der Testumgebung gemacht. Eine Überprüfung zur Laufzeit findet derzeit nicht statt.

Es ist daher eine Aufgabe eine verbesserte medizinische Vorrichtung, ein verbessertes medizinisches System und ein verbessertes Verfahren zur Überwachung eines Quell-Bildstroms mit mehreren aufeinanderfolgenden Quell-Bildern aufzuzeigen, die auch eine kontinuierliche Überwachung zur Laufzeit ermöglichen.

Die Aufgabe wird nach einem ersten Aspekt gelöst durch eine medizinische Vorrichtung zur Überwachung eines Quell-Bildstroms mit mehreren aufeinanderfolgenden Quell-Bildern, wobei die Vorrichtung aufweist:
eine Verarbeitungseinrichtung, die dafür ausgebildet ist, den Quell-Bildstrom zu empfangen, in den Quell-Bildern für mehrere vorgegebene Bereiche eines jeweiligen Quell-Bilds einen jeweiligen Sicherungswert zu errechnen, basierend auf den Sicherungswerten der mehreren Bereiche eine Sicherungsinformation zu bestimmen, dem jeweiligen Quell-Bild die Sicherungsinformation hinzuzufügen und die daraus resultierenden Bilder als Sende-Bilder eines Sende-Bildstroms zu senden;
eine Verifikationseinrichtung, die dafür ausgebildet ist, den Sende-Bildstrom zu empfangen, für die mehreren vorgegebenen Bereiche eines jeweiligen Sende-Bilds einen jeweiligen Prüfwert zu errechnen, die Sicherungswerte der mehreren Bereiche aus der Sicherungsinformation aus einem jeweiligen Sende-Bild zu extrahieren und ein Warnsignal auszugeben, falls eine Abweichung zwischen den Sicherungswerten und den Prüfwerten einen vorgegebenen Schwellwert überschreitet,
wobei die Sicherungswerte und die Prüfwerte jeweils basierend auf numerischen Farbinformationen von Pixeln berechnet werden, die innerhalb eines jeweiligen Bereichs liegen.

Diese Vorrichtung ermöglicht eine vorteilhafte Überwachung dahingehend, dass der Sende-Bildstrom, der am Ende eines Übertragungsweges von einem Benutzer wahrgenommen wird, hinreichend genau dem ursprünglich generierten Quell-Bildstrom entspricht. Ist dies nicht der Fall wird ein Warnsignal ausgegeben. Das Warnsignal kann automatisiert verarbeitet werden, zum Beispiel indem auf einen anderen Übertragungskanal zwischen der Verarbeitungseinrichtung und der Verifikationseinrichtung umgeschaltet wird, indem eine Datenreduktion verringert wird oder der Quell-Bildstrom mit mehr redundanten Daten gesendet wird.

Die Bereiche werden bevorzugt als einfache geometrische Formen festgelegt, insbesondere als Rechtecke. Es kann aber je nach Anwendungsfall auch vorteilhaft sein, die Bereiche als Bildschablone zu definieren. Entscheidend ist bei der Wahl der Bereiche, dass sie so gewählt werden, dass Abweichungen zwischen Bereichen des Quell-Bildstroms und den entsprechenden Bereichen des Sende-Bildstroms, die für die Fachperson relevant sind oder sein könnten, zuverlässig automatisch erkannt werden können. Daher ist es bevorzugt, dass ein einzelner Bereich mindestens 0,1% der Gesamtfläche eines Quell-Bilds erfasst und/oder höchstens 5%, 2% oder 1 % dieser Gesamtfläche erfasst. Zudem ist es bevorzugt, dass eine Gesamtheit der mehreren vorgegebenen Bereiche weniger als 100% eines Quell-Bilds erfasst, besonders bevorzugt weniger als 75% oder weniger als 50% und insbesondere weniger als 25% oder weniger als 10%.

Unter den numerischen Farbinformationen sind bevorzugt Farbwert, Farbsättigung und/oder Hellwert bzw. Luminanz zu verstehen. Diese können unmittelbar vorgegeben werden oder sich aus den Einzelwerten des jeweiligen Farbmodells ergeben, also zum Beispiel aus den Rot-, Grün und Blau-Anteilen beim RGB-Farbmodell. Die Berechnung kann insbesondere einen Mittelwert, einen Median, einen gewichteten Mittelwert, einen geometrischen Mittelwert, eine Maximalwert oder einen Minimalwert ermitteln.

Zur Ausgabe des Warnsignals weist die Vorrichtung bevorzugt eine Ausgabevorrichtung auf, die dafür ausgebildet ist, das Warnsignal visuell, akustisch und/oder haptisch erfassbar auszugeben. Bei dieser Ausgestaltung erhält der Benutzer einer Hinweis, wenn der Ist-Sende-Bildstrom von dem erwarteten Soll-Sende-Bildstrom abweicht. Der Benutzer ist dann dafür sensibilisiert, dass Ist-Sende-Bildstrom evtl. nicht das zeigt, was der Benutzer bei einer idealen, fehlerfreien Übertragung erwarten würde. Das Warnsignal kann auch Informationen über die Art der Warnung, sprich, über die Art und Schwere der nicht erfüllten Bedingung oder Bedingungen enthalten. Es ist dann möglich, dass der Benutzer auswählen kann, ob und wie er in den jeweiligen Abweichungen vom gewünschten Soll-Sende-Bildstrom benachrichtigt werden will.

Als Schwellwert kann insbesondere definiert werden, dass bezogen auf ein Sende-Bild eine definierte Anzahl von Bereichen, entweder ausgedrückt als absolute Anzahl oder als relativer Prozentsatz von der Gesamtzahl der Bereiche, bei denen der Prüfwert außerhalb eines definierten Toleranzbereichs um den Sicherungswert liegt nicht überschritten werden soll. Ein solcher Toleranzbereich kann insbesondere als +/- 0, +/- 1%, +/- 2%, +/-5% oder +/- 10% bezogen auf den maximalen Wertebereich des Sicherungswerts gewählt werden. Der Toleranzbereich wird bevorzugt für Bereiche nahe der Bildmitte enger gewählt als in von der Bildmitte entfernten Bereichen.

Eine Besonderheit dieses Ansatzes liegt darin, dass eine ordnungsgemäße Übertragung der Quell-Bilder überwacht werden kann, ohne eine Identität zwischen den Quell-Bildern und den Sende-Bildern zu fordern, wie man sie zum Beispiel über eine Prüfsumme über alle Bildpixel ermitteln könnte. Stattdessen berücksichtigt dieser Ansatz, dass die Quell-Bilder durchaus in Details von den Sende-Bildern abweichen können, ohne dass dies aus visueller, humanphysiologischer Sicht relevant ist. Zudem kann durch eine geeignete Wahl der Bereiche bewirkt werden, dass die Übereinstimmung in einigen Teile der Quell-Bilder als wichtiger erachtet wird als in anderen Teilen.

Damit ist die Aufgabe vollständig gelöst.

Bei einer bevorzugten Ausgestaltung sind die mehreren Bereiche nahe einer Bildmitte der Quell-Bilder kleiner, als wenn sie entfernt von der Bildmitte sind.

Diese Ausgestaltung ermöglicht es zu berücksichtigen, dass Informationen in der Bildmitte in aller Regel für die Wahrnehmung durch eine Fachperson einen höheren Stellenwert haben als Informationen am Bildrand. Die Größe eines Bereichs wird dabei insbesondere im Hinblick auf die in diesem Bereich enthaltenen Bildpixel verstanden.

Bei einer weiteren bevorzugten Ausgestaltung ist eine Größe der mehreren Bereiche proportional zu einer Entfernung von einer Bildmitte der Quell-Bilder.

Diese Ausgestaltung ermöglicht eine einfache Bestimmung der geeigneten Größe der Bereiche. Die Proportionalität kann insbesondere linear, logarithmisch oder exponentiell sein.

Bei einer weiteren bevorzugten Ausgestaltung überlagern sich mindestens zwei der mehreren Bereiche.

Diese Ausgestaltung ermöglicht es, dass die Schnittmenge zwischen den mindestens zwei Bereichen mehrfach bewertet wird, jeweils in Verbindung mit einer unterschiedlichen komplementären Menge, die innerhalb eines jeweiligen Bereichs aber außerhalb der Schnittmenge liegt. Dadurch können mögliche Artefakte bei der Bewertung reduziert oder unterdrückt werden, die sich in besonderen Bildsituationen ergeben können.

Bei einer weiteren bevorzugten Ausgestaltung sind Zentren der mehreren Bereiche entlang einer Spiralform angeordnet.

Diese Ausgestaltung ermöglicht eine wohldefinierte Abdeckung eines Quell-Bilds, mit der in Abhängigkeit von der gewählten Spiralform und der gewählten Größe der Bereiche das gesamte Quell-Bild abgedeckt werden kann. Das Zentrum eines Bereichs kann als insbesondere als geometrischer Mittelpunkt oder Schwerpunkt verstanden werden. Die Spiralform stellt sicher, dass kein einheitliches Raster über alle Bereiche entsteht. Dadurch können mögliche Artefakte bei der Bewertung reduziert oder unterdrückt werden. Die Zentren können entlang der Spirale in definierten Abständen angeordnet sein. Diese Abstände können sich im Verlauf der Spirale fortlaufend vergrößern.

Bei einer weiteren bevorzugten Ausgestaltung ist die Spiralform eine archimedische Spirale.

Die archimedische Spirale lässt sich im kartesischen Koordinatensystem mit den Formeln x = k * phi * cos(phi) und y = k * phi * sin(phi) beschreiben, wobei phi als Winkel verstanden werden kann und kein Streckungs- bzw. Stauchungsfaktor für die Spirale ist. Umgekehrt gilt dann auch phi = atan(y / x). Die archimedische Spirale hat die Eigenschaft, dass jede Gerade, die durch ihren Ursprungspunkt verläuft, die Spirale mehrfach schneidet, wobei diese Schnittpunkte entlang der Gerade gleichweit voneinander entfernt sind. Es lässt sich eine gute Verteilung der Bereiche erzielen, wenn diese Schnittpunkte als Mittelpunkte der Bereiche gewählt werden.

Bei einer weiteren bevorzugten Ausgestaltung dreht sich die Spiralform im zeitlichen Verlauf der Quell-Bilder gemäß einer definierten Vorgabe fortlaufend.

Bei dieser Ausgestaltung werden fortlaufend andere Bereiche der Quell-Bilder erfasst. Auf diese Weise kann es ausreichend sein, immer nur Teile der Quell-Bilder in die Sicherungsinformation aufzunehmen, da durch die Drehung der Spirale, insbesondere nach innen, und die darauf resultierende Veränderung der Bereiche eine ausreichende Überprüfung stattfindet. Da die Spiralform vorgegeben wird und auch die Anordnung der Bereiche entlang der Spiralform einfach beschrieben werden kann, z.B. durch Längen entlang der Spiralform oder durch eine Winkeländerung beim Winkel phi, kann die Verifikationseinrichtung selbsttätig die Position und Größe der Bereiche bestimmen, die überprüft werden sollen, sprich, die Bereiche, für die die Verarbeitungseinrichtung die Sicherungswerte bestimmt hat.

Die Spiralform wird so gewählt, dass die genannten Bereiche im Laufe der Zeit jeden Teil des Bildes, oder zumindest jeden Bildbereich von Interesse (ROI, region of interest) überstreichen. So kann erreicht werden, dass Bildteile, welche aktuell nicht in den Bereichen liegen, in den nachfolgenden Bildsequenzen berücksichtigt werden. Damit ist das Verfahren nicht nur eine Einzelbildprüfung, sondern auch eine kontinuierliche Bildsequenzprüfung - die Genauigkeit oder Zuverlässigkeit steigt mit zunehmender Laufzeit. Dies hat den Vorteil, dass die Prüfbereiche pro Quell-Bild reduziert, also weniger und/oder kleiner sein können.

Bei einer weiteren bevorzugten Ausgestaltung ist die Verarbeitungseinrichtung ferner dafür ausgebildet, die Sicherungsinformation in mehrere Sicherungspixel umzurechnen und die Sicherungspixel als die Sicherungsinformation dem Quell-Bild hinzuzufügen, und die Verifikationseinrichtung ist ferner dafür ausgebildet, die in den mehreren Sicherungspixeln enthaltene Sicherungsinformation zu extrahieren.

Diese Ausgestaltung ermöglicht einen besonders einfachen Transport der Sicherungsinformation. Das Hinzufügen der Sicherungspixel erfolgt dabei bevorzugt durch Überschreiben von Bildinformation im Quell-Bild. Die Sicherungsinformation kann beispielsweise als Binärcode von schwarzen und weißen Sicherungspixeln abgebildet werden.

Bei einer weiteren bevorzugten Ausgestaltung sind die Sicherungspixel in einem Randbereich der Sende-Bilder angeordnet, insbesondere entlang eines Rands der Sende-Bilder.

Diese Ausgestaltung ermöglicht es die Sicherungspixel auf einer Weise zu transportieren, die den Benutzer nicht stört oder nahezu nicht wahrnehmbar ist. Insbesondere wird die Sicherungsinformation außerhalb des Bildmotivs angeordnet, also z.B. in oder bei einem Rahmen aufgeprägt, so dass der Betrachter dies nicht als störend empfindet.

Bei einer weiteren bevorzugten Ausgestaltung bilden die Sicherungspixel einen maschinen-lesbaren Code, insbesondere einen QR-Code.

Diese Ausgestaltung ermöglicht es die Sicherungsinformation auf einfache standardisierte Weise auszulesen. Dies ist sogar dann möglich, wenn das Sende-Bild nur noch als solches verfügbar ist, z.B. wenn es nur noch als Ausdruck vorliegt.

Bei einer weiteren bevorzugten Ausgestaltung wird aus den numerischen Farbinformationen der Pixel eine durchschnittliche Luminanz innerhalb eines Bereichs ermittelt und der Sicherungswert und der Prüfwert sind jeweils die durchschnittliche Luminanz.

Diese Ausgestaltung hat sich in praktischen Versuchen als besonders geeignet erwiesen.

Bei einer weiteren bevorzugten Ausgestaltung weist die Sicherungsinformation zusätzlich eine Positionsinformation zu jedem der mehreren Bereiche auf.

Diese Ausgestaltung ermöglicht es der Verifikationseinrichtung die Bereiche zu identifizieren, die von der Verarbeitungseinrichtung der Bestimmung der Sicherungswerte zugrunde gelegt wurden. Es wird bei etlichen Ausgestaltungen zwar bevorzugt sein, dass die Verifikationseinrichtung die von der Verarbeitungseinrichtung verwendeten Bereiche kennt oder diese Bereiche z.B. über eine mathematische Formel selbsttätig bestimmen kann. Bei dieser Ausgestaltung muss ein solches Wissen aber nicht der Verifikationseinrichtung zur Verfügung gestellt werden, da die Verifikationseinrichtung die Positionsinformation zur Bestimmung der Bereiche heranzieht. Als Positionsinformation können beispielsweise alle Eckpunkte eines Bereichs, zwei gegenüberliegende Ecken eines Rechtecks, ein Mittelpunkt mit mindestens einer weiteren Größenangabe, usw. gewählt werden.

Es sei darauf hingewiesen, dass als Positionsinformation zu jedem der mehreren Bereiche auch als die zuvor genannte mathematische Formel übermittelt werden kann oder als jede andere programmatische Anweisung mittels derer sich die Bereiche bestimmen lassen. Aus der mathematischen Formel, wie die Bereiche angeordnet sind, oder der programmatischen Anweisung kann dann die konkrete Information zu den einzelnen Bereichen ermittelt werden.

Gemäß einem zweiten Aspekt wird die Aufgabe gelöst durch ein medizinisches System zur Überwachung eines Bildstroms mit mehreren aufeinanderfolgenden Bildern aufweisend einen Bildgeber zur Erzeugung des Quell-Bildstroms, einer Vorrichtung nach einem der vorhergehenden Ansprüche, und einer Anzeigevorrichtung, die dafür ausgebildet ist, den Sende-Bildstrom auszugeben und das Warnsignal wiederzugeben, wobei die Verarbeitungseinrichtung insbesondere in den Bildgeber integriert ist.

Gemäß einem dritten Aspekt wird die Aufgabe gelöst durch ein Verfahren zur Überwachung eines Quell-Bildstroms mit mehreren aufeinanderfolgenden Quell-Bildern, das Verfahren mit den Schritten:
Empfangen des Quell-Bildstroms;
Errechnen, in den Quell-Bildern für mehrere vorgegebene Bereiche eines jeweiligen Quell-Bilds, eines jeweiligen Sicherungswerts;
Bestimmen, basierend auf den Sicherungswerten der mehreren Bereiche, einer Sicherungsinformation;
Hinzufügen der Sicherungsinformation zu dem jeweiligen Quell-Bild;
Senden der daraus resultierenden Bilder als Sende-Bilder eines Sende-Bildstroms;
Empfangen des Sende-Bildstroms;
Errechnen eines jeweiligen Prüfwerts für die mehreren vorgegebenen Bereiche eines jeweiligen Sende-Bilds;
Extrahieren der Sicherungswerte der mehreren Bereiche aus der Sicherungsinformation aus einem jeweiligen Sende-Bild; und
Ausgeben eines Warnsignals, falls eine Abweichung zwischen den Sicherungswerten und den Prüfwerten einen vorgegebenen Schwellwert überschreitet,
wobei die Sicherungswerte und die Prüfwerte jeweils basierend auf numerischen Farbinformationen von Pixeln berechnet werden, die innerhalb eines jeweiligen Bereichs liegen.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1: eine Ausführungsform eines medizinisches Systems zur Überwachung eines Quell-Bildstroms mit mehreren aufeinanderfolgenden Quell-Bildern mit einer Ausführungsform einer medizinisches Vorrichtung zur Überwachung eines Quell-Bildstroms mit mehreren aufeinanderfolgenden Quell-Bildern;
- Fig. 2: eine Ausführungsform eines Verfahrens zur Überwachung eines Quell-Bildstroms mit mehreren aufeinanderfolgenden Quell-Bildern;
- Fig. 3: eine Ausführungsform eines Quell-Bilds;
- Fig. 4: eine erste Ausführungsform eines Sende-Bilds;
- Fig. 5: eine Ausführungsform einer Spiralform; und
- Fig. 6: eine zweite Ausführungsform eines Sende-Bilds.

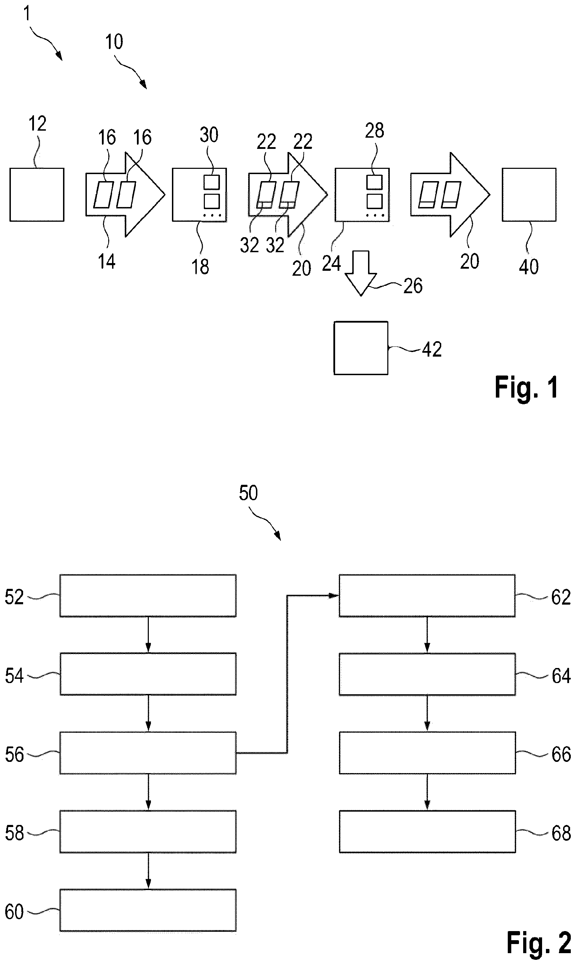

Fig. 1 zeigt ein medizinisches System 1 zur Überwachung eines Quell-Bildstroms 14 mit mehreren aufeinanderfolgenden Quell-Bildern 16 aufweisend einen Bildgeber 12 zur Erzeugung des Quell-Bildstroms 14, einer medizinischen Vorrichtung 10 zur Überwachung eines Quell-Bildstroms 14 mit mehreren aufeinanderfolgenden Quell-Bildern 16, und einer Anzeigevorrichtung 40, die dafür ausgebildet ist, den Sende-Bildstrom auszugeben und das Warnsignal 26 wiederzugeben.

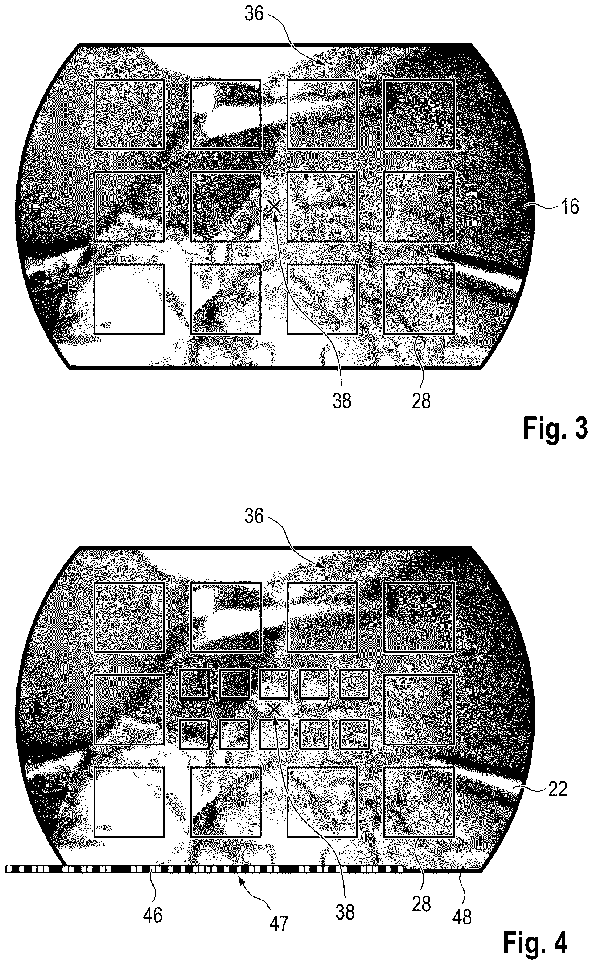

Die Vorrichtung 10 weist verschiedene Elemente auf, darunter eine Verarbeitungseinrichtung 18, die dafür ausgebildet ist, den Quell-Bildstrom 14 zu empfangen und in den Quell-Bildern 16 für mehrere vorgegebene Bereiche 30 (siehe Fig. 3) eines jeweiligen Quell-Bilds 16 einen jeweiligen Sicherungswert 30 zu errechnen. Die Berechnung des Sicherungswert 30 wir nachfolgend noch erläutert.

Basierend auf den Sicherungswerten 30 der mehreren Bereiche 28 bestimmt die Verarbeitungseinrichtung 18 dann eine Sicherungsinformation 32 und fügt die Sicherungsinformation 32 dem jeweiligen Quell-Bild 16 hinzu. Die daraus resultierenden Bilder sendet die Verarbeitungseinrichtung 18 dann als Sende-Bilder 22 eines Sende-Bildstroms 20.

Die Vorrichtung 10 weist ferner eine Verifikationseinrichtung 24 auf, die dafür ausgebildet ist, den Sende-Bildstrom 20 zu empfangen und für die mehreren vorgegebenen Bereiche 28 eines jeweiligen Sende-Bilds 22 einen jeweiligen Prüfwert 34 zu errechnen. Die Verifikationseinrichtung 24 extrahiert außerdem die Sicherungswerte 30 der mehreren Bereiche 28 aus der Sicherungsinformation 32 aus einem jeweiligen Sende-Bild 22 und gibt ein Warnsignal 26 aus, falls eine Abweichung zwischen den Sicherungswerten 30 und den Prüfwerten 34 einen vorgegebenen Schwellwert überschreitet.

Die Sicherungswerte 30 und die Prüfwerte 34 werden jeweils basierend auf numerischen Farbinformationen von Pixeln 36 berechnet, die innerhalb eines jeweiligen Bereichs 28 liegen. Auf diese Weise werden für Bereiche, die zwischen einem Quell-Bild 16 und einem entsprechenden Sende-Bild 22 übereinstimmen jeweils charakteristische Werte berechnet, die zwischen dem Quell-Bild 16 und seinem entsprechenden Sende-Bild 22 übereinstimmen sollten, zumindest im Rahmen einer vorgegebenen Toleranz.

Der Schwellwert, der festlegt, ob ein Warnsignal 26 ausgegeben werden soll oder nicht, kann sowohl die Größe von einer oder mehreren Abweichungen zwischen Quell-Bildern 16 und ihren entsprechenden Sende-Bildern 22 berücksichtigen als auch die Anzahl der Abweichungen, die zwischen Quell-Bildern 16 und ihren entsprechenden Sende-Bildern 22 auftreten. So kann insbesondere festgelegt werden, dass die Größe einer einzelnen Abweichung einen ersten Betrag nicht übersteigen darf und/oder die Anzahl der Abweichungen einen zweiten Betrag nicht übersteigen darf. Dabei ist es bevorzugt, dass eine Gewichtung bei den Abweichungen vorgenommen wird, wobei wenige große Abweichungen als problematischer angesehen werden als mehrere kleine Abweichungen.

Fig. 2 zeigt ein Verfahren 50 zur Überwachung eines Quell-Bildstroms 14 mit mehreren aufeinanderfolgenden Quell-Bildern 16. Die Ausführungsform des gezeigten Verfahrens 50 beginnt mit dem Empfangen 52 des Quell-Bildstroms 14 gefolgt von einem Errechnen 54, in den Quell-Bildern 16 für mehrere vorgegebene Bereiche 28 eines jeweiligen Quell-Bilds 16, eines jeweiligen Sicherungswerts 30. Diese Berechnung findet wie bereits beschrieben statt.

In einem weiteren Schritt erfolgt ein Bestimmen 56, basierend auf den Sicherungswerten 30 der mehreren Bereiche, einer Sicherungsinformation 32 und ein Hinzufügen 58 der Sicherungsinformation 32 zu dem jeweiligen Quell-Bild 16. Anschließend erfolgt das Senden 60 der daraus resultierenden Bilder als Sende-Bilder 22 eines Sende-Bildstroms 20. Das Bestimmen 56 der Sicherungsinformation 32 kann bei einer Ausgestaltung eine durch Trennzeichen getrennte Aneinanderreihung der Sicherungswerte 30 sein.

Nachdem die Sende-Bilder 22 des Sende-Bildstroms 20 eine Übertragungsstrecke zurückgelegt haben, insbesondere über ein Gerätekabel und/oder ein Netzwerk, erfolgt an einem Zielpunkt ein Empfangen 62 des Sende-Bildstroms 20 und ein Errechnen 64 eines jeweiligen Prüfwerts 34 für die mehreren vorgegebenen Bereiche 28 eines jeweiligen Sende-Bilds 22.

Um die Prüfwerte 34 mit einem Sollwert abgleichen zu können findet ein Extrahieren 66 der Sicherungswerte 30 der mehreren Bereiche aus der Sicherungsinformation 32 aus einem jeweiligen Sende-Bild 22 statt und ein Ausgeben 68 eines Warnsignals 26, falls eine Abweichung zwischen den Sicherungswerten 30 und den Prüfwerten 34 einen vorgegebenen Schwellwert überschreitet.

Auch hier gilt wieder, dass die Sicherungswerte 30 und die Prüfwerte 34 jeweils basierend auf numerischen Farbinformationen von Pixeln 36 berechnet werden, die innerhalb eines jeweiligen Bereichs 28 liegen.

Fig. 3 zeigt eine Ausführungsform eines Quell-Bilds 16 mit einer ersten Ausführungsform mit zwölf Bereichen 28. Zum Zwecke einer besseren Übersichtlichkeit wurde nur ein Bereich 28 mit einem Bezugszeichen versehen. Die Bereiche 28 sind hier und im Nachfolgenden nur für ein besseres Verständnis dargestellt. Sie sind für den Benutzer grundsätzlich nicht sichtbar. Bei bevorzugten Ausführungsformen werden jedoch solche Bereiche 28 dargestellt, insbesondere mit einem farbigen Rahmen, in denen größere Abweichungen zwischen dem Sicherungswert und dem Prüfwert festgestellt werden. Es ist hier außerdem eine Bildmitte 38 gekennzeichnet.

Fig. 4 zeigt eine erste Ausführungsform eines Sende-Bilds 22 basierend auf dem Quell-Bild 16 aus Fig. 3 mit einer zweiten Ausführungsform mit 20 Bereichen 28. Zum Zwecke einer besseren Übersichtlichkeit wurde auch hier wieder nur ein Bereich 28 mit einem Bezugszeichen versehen.

Zusätzlich ist hier nun eine Wiedergabe der Sicherungsinformation 32 als Sicherungspixel 46 in einem Randbereich 47 des Sende-Bilds 22 gezeigt, konkret an einem Rand 48 des Sende-Bilds 22.

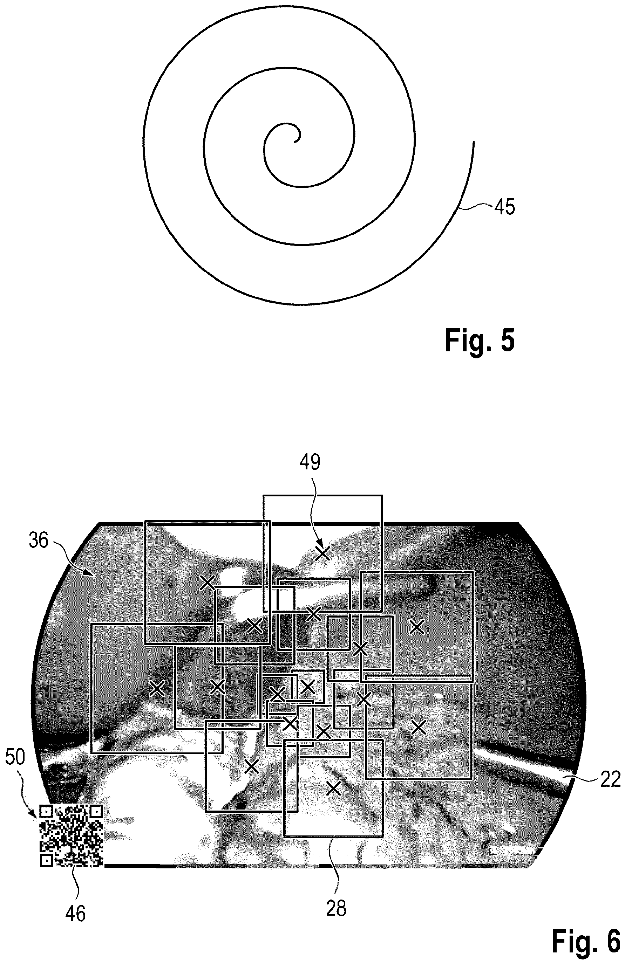

Fig. 5 zeigt eine Ausführungsform einer Spiralform 45. Die Spiralform 45 ist hier eine archimedische Spirale.

Fig. 6 zeigt eine Ausführungsform eine zweite Ausführungsform eines Sende-Bilds 22 basierend auf dem Quell-Bild 16 aus Fig. 3 mit einer dritten Ausführungsform von Bereichen 28, die hier entlang der in Fig. 5 gezeigten Spiralform 45 angeordnet sind. Konkret sind Mittelpunkte bzw. Zentren 49 der quadratischen Bereiche 28 entlang der Spiralform 45 angeordnet. Zum Zwecke einer besseren Übersichtlichkeit wurde nur ein Mittelpunkt 49 mit einem Bezugszeichen versehen.

Die Mittelpunkte 49 sind hier um einen Winkel von ca. 360°/7 voneinander beanstandet. Außerdem steigt die Größe der quadratischen Bereiche 28 ausgehend von der Bildmitte 38 an.

Im Unterschied zur Fig. 4 erfolgt hier eine Wiedergabe der Sicherungsinformation 32 als Sicherungspixel 46 in der Form eines maschinen-lesbaren Code 50.

## Patentansprüche

1. Medizinische Vorrichtung (10) zur Überwachung eines Quell-Bildstroms (14) mit mehreren aufeinanderfolgenden Quell-Bildern (16), wobei die Vorrichtung (10) aufweist:
eine Verarbeitungseinrichtung (18), die dafür ausgebildet ist, den Quell-Bildstrom (14) zu empfangen, in den Quell-Bildern (16) für mehrere vorgegebene Bereiche eines (30) jeweiligen Quell-Bilds (16) einen jeweiligen Sicherungswert (30) zu errechnen, basierend auf den Sicherungswerten (30) der mehreren Bereiche (28) eine Sicherungsinformation (32) zu bestimmen, dem jeweiligen Quell-Bild (16) die Sicherungsinformation (32) hinzuzufügen und die daraus resultierenden Bilder als Sende-Bilder (22) eines Sende-Bildstroms (20) zu senden;
eine Verifikationseinrichtung (24), die dafür ausgebildet ist, den Sende-Bildstrom (20) zu empfangen, für die mehreren vorgegebenen Bereiche (28) eines jeweiligen Sende-Bilds (22) einen jeweiligen Prüfwert (34) zu errechnen, die Sicherungswerte (30) der mehreren Bereiche (28) aus der Sicherungsinformation (32) aus einem jeweiligen Sende-Bild (22) zu extrahieren und ein Warnsignal (26) auszugeben, falls eine Abweichung zwischen den Sicherungswerten (30) und den Prüfwerten (34) einen vorgegebenen Schwellwert überschreitet,
wobei die Sicherungswerte (30) und die Prüfwerte (34) jeweils basierend auf numerischen Farbinformationen von Pixeln (36) berechnet werden, die innerhalb eines jeweiligen Bereichs (28) liegen.

2. Medizinische Vorrichtung (10) nach Anspruch 1, wobei die mehreren Bereiche (28) nahe einer Bildmitte (38) der Quell-Bilder (16) kleiner sind, als wenn sie entfernt von der Bildmitte (38) sind.

3. Medizinische Vorrichtung (10) nach einer der vorhergehenden Ansprüche, wobei eine Größe der mehreren Bereiche (28) proportional zu einer Entfernung von einer Bildmitte (38) der Quell-Bilder (16) ist.

4. Medizinische Vorrichtung (10) nach einer der vorhergehenden Ansprüche, wobei sich mindestens zwei der mehreren Bereiche (28) überlagern.

5. Medizinische Vorrichtung (10) nach einer der vorhergehenden Ansprüche, wobei Zentren (49) der mehreren Bereiche (28) entlang einer Spiralform (45) angeordnet sind.

6. Medizinische Vorrichtung (10) nach Anspruch 5, wobei die Spiralform (45) eine archimedische Spirale ist.

7. Medizinische Vorrichtung (10) nach einem der Ansprüche 5 oder 6, wobei sich die Spiralform (45) im zeitlichen Verlauf der Quell-Bilder (16) gemäß einer definierten Vorgabe fortlaufend dreht.

8. Medizinische Vorrichtung (10) nach einer der vorhergehenden Ansprüche, wobei die Verarbeitungseinrichtung (18) ferner dafür ausgebildet ist, die Sicherungsinformation (32) in mehrere Sicherungspixel (46) umzurechnen und die Sicherungspixel (46) als die Sicherungsinformation (32) dem Quell-Bild (16) hinzuzufügen, und die Verifikationseinrichtung (24) ferner dafür ausgebildet ist, die in den mehreren Sicherungspixeln (46) enthaltene Sicherungsinformation (32) zu extrahieren.

9. Medizinische Vorrichtung (10) nach Anspruch 8, wobei die Sicherungspixel in einem Randbereich (47) der Sende-Bilder (22) angeordnet sind, insbesondere entlang eines Rands (48) der Sende-Bilder (22).

10. Medizinische Vorrichtung (10) nach Anspruch 8, wobei die Sicherungspixel (46) einen maschinen-lesbaren Code (50) bilden, insbesondere einen QR-Code.

11. Medizinische Vorrichtung (10) nach einer der vorhergehenden Ansprüche, wobei aus den numerischen Farbinformationen der Pixel (36) eine durchschnittliche Luminanz innerhalb eines Bereichs (28) ermittelt wird und die Sicherungswerte (30) und die Prüfwerte (34) jeweils die durchschnittliche Luminanz sind.

12. Medizinische Vorrichtung (10) nach einer der vorhergehenden Ansprüche, wobei die Sicherungsinformation (32) zusätzlich eine Positionsinformation zu jedem der mehreren Bereiche (28) aufweist.

13. Medizinische System (1) zur Überwachung eines Quell-Bildstroms (14) mit mehreren aufeinanderfolgenden Quell-Bildern (16) aufweisend einen Bildgeber (12) zur Erzeugung des Quell-Bildstroms (14), einer Vorrichtung (10) nach einem der vorhergehenden Ansprüche, und einer Anzeigevorrichtung (40), die dafür ausgebildet ist, den Sende-Bildstrom (20) auszugeben und das Warnsignal (26) wiederzugeben, wobei die Verarbeitungseinrichtung (18) insbesondere in den Bildgeber (12) integriert ist.

14. Verfahren (50) zur Überwachung eines Quell-Bildstroms (14) mit mehreren aufeinanderfolgenden Quell-Bildern (16), das Verfahren mit den Schritten:
Empfangen (52) des Quell-Bildstroms (14);
Errechnen (54), in den Quell-Bildern (16) für mehrere vorgegebene Bereiche (28) eines jeweiligen Quell-Bilds (16), eines jeweiligen Sicherungswerts (30);
Bestimmen (56), basierend auf den Sicherungswerten (30) der mehreren Bereiche, einer Sicherungsinformation (32);
Hinzufügen (58) der Sicherungsinformation (32) zu dem jeweiligen Quell-Bild (16);
Senden (60) der daraus resultierenden Bilder als Sende-Bilder (22) eines Sende-Bildstroms (20);
Empfangen (62) des Sende-Bildstroms (20);
Errechnen (64) eines jeweiligen Prüfwerts (34) für die mehreren vorgegebenen Bereiche (28) eines jeweiligen Sende-Bilds (22);
Extrahieren (66) der Sicherungswerte (30) der mehreren Bereiche aus der Sicherungsinformation (32) aus einem jeweiligen Sende-Bild (22); und
Ausgeben (68) eines Warnsignals (26), falls eine Abweichung zwischen den Sicherungswerten (30) und den Prüfwerten (34) einen vorgegebenen Schwellwert überschreitet,
wobei die Sicherungswerte (30) und die Prüfwerte (34) jeweils basierend auf numerischen Farbinformationen von Pixeln (36) berechnet werden, die innerhalb eines jeweiligen Bereichs (28) liegen.
